# EUROPEAN PATENT SPECIFICATION

(11) **EP 2 719 372 B1**
(45) Date of publication and mention of the grant of the patent: **20.02.2019**
(21) Application number: 12800589.9
(22) Date of filing: 31.05.2012
(51) Int. Cl.: A61K 8/02, A61K 8/06, A61K 8/19, A61K 8/20, A61K 8/46, A61Q 1/02

(54) **EMULSION-TYPE MAKE-UP BASE AND METHOD FOR PRODUCING SAME**
MAKE-UP-BASISSTOFF IN EMULSIONSFORM UND HERSTELLUNGSVERFAHREN DAFÜR
BASE DE MAQUILLAGE DE TYPE ÉMULSION ET SON PROCÉDÉ DE PRODUCTION

(30) Priority: 13.06.2011 JP 2011131540; 30.05.2012 JP 2012123428
(43) Date of publication of application: 16.04.2014
(73) Proprietor: Fujifilm Corporation, Minato-ku Tokyo 106-8620 (JP)
(72) Inventor: AIMI, Makiko, Ashigarakami-gun Kanagawa 258-8577 (JP); KINAI, Miki, Ashigarakami-gun Kanagawa 258-8577 (JP); NAKAMURA, Kazuhiro, Ashigarakami-gun Kanagawa 258-8577 (JP)
(74) Representative: Klunker IP Patentanwälte PartG mbB
(86) International application number: PCT/JP2012/064202
(87) International publication number: WO 2012/173000

(56) References cited:
- JP-A- 7 304 633
- JP-A- 11 012 493
- JP-A- 11 035 840
- JP-A- 2002 220 320
- JP-A- 2003 105 225
- JP-A- 2008 222 586
- US-A1- 2003 003 065

## Description

### Technical Field

The present invention relates to an emulsion-type base makeup cosmetic and a method of producing the same.

### Background Art

A base makeup cosmetic such as a foundation is required to have the ability to cover up dullness in skin color (a state with lowered lightness and increased yellow chroma) which is perceived with aging caused by poor blood circulation or pigmentation, and to give a natural finished color tone or the like; and various base makeup cosmetics and methods of production thereof have been proposed.

For example, as a technique focused on realization of a natural and healthy skin color without dullness, a foundation having a depression in a spectrum in a wavelength region of from 500 to 620 nm in a reflectometric spectrum has been disclosed by Japanese Patent No. 3514915.

A colorant used for a cosmetic and the like having various capabilities simultaneously has been proposed and, for example, Japanese Patent Application Laid-Open (JP-A) No. 2003-105225 discloses a technique in which an organic composite pigment, in which an organic dye stuff is fixed to an inorganic substance, is added in view of improvement in color development and suppression of staining of skin.

On the other hand, it is necessary to add a larger amount of a surfactant or a special surfactant to provide a highly stable emulsion-type base makeup cosmetic. For example, JP-A No. 2010-111634 discloses a technique for adding an amphiphilic polymer. However, addition of an increased amount of a surfactant to a base makeup cosmetic leads to an unpleasant skin sensation such as tackiness.
JP 2008-222586 A teaches a water-in-oil type emulsion cosmetic obtained by using an ultramarine blue pigment of an inorganic pigment exhibiting a vivid color of pink to blue, and having excellent temporal stability as viscosity change. The water-in-oil type emulsion cosmetic contains (A) a divalent metal salt and (B) the ultramarine blue pigment surface-treated with silicones. Preferably, the cosmetic contains one or more kinds selected from magnesium sulfate, magnesium chloride and calcium chloride as the component (A), and the ultramarine blue pigment.
US 2003/003065 A1 teaches cosmetic compositions and cosmetic compositions that have been adapted for delivery to provide applied cosmetic compositions that have a spectrophotometric curve, wherein a first derivative of the spectrophotometric curve comprises: a) a maximum peak in the region of from about 430 nm to about 520 nm occurs at a wavelength not greater than about 480 nm; b) a maximum peak in the region of from about 420 nm to about 650 nm occurs at a wavelength of from about 570 nm to about 630 nm; and c) a minimum valley in the region of from about 520 nm to about 580 nm and has a Δ%R/Δλ of less than or equal to about 0.03, wherein R is reflectance and λ is the wavelength, and wherein the cosmetic composition comprises a mixture of at least two colorants, wherein a first derivative of a spectrophotometric curve of each of the individual colorants does not exhibit (a), (b) and (c).
JP 11 035840 A discloses cosmetic compositions. However, JP 11 035840 A fails to disclose a powdered pigment comprising a red composite pigment in which lithol rubine BCA is intercalated into an inorganic substance and further fails to teach a cosmetic composition comprising a water-soluble calcium salt which is at least one selected from calcium chloride, calcium lactate, or calcium acetate.

### SUMMARY OF INVENTION

### Technical Problem

Conventional base makeup cosmetics have a problem in that the color tone of the skin color may appear yellowish (hereinafter also referred to as "yellow dulling") depending on the light source when the base makeup cosmetic is applied to the skin, and thus there is a drawback in that a natural skin color finish that is independent of a light source cannot be obtained.

Further, with respect to an emulsion-type base makeup cosmetic, there is a drawback that a pigment included in a cosmetic base material as a colorant dissolves into the solvent (bleeds out) causing uneven coloring and impairing the appearance of the cosmetic, or staining when applied to the skin

Furthermore, addition of an increased amount of a surfactant to improve the stability of an emulsion-type base makeup cosmetic may cause tackiness. Use of novel additives may also raise safety concerns.

Additionally, with respect to an emulsion-type base makeup cosmetic, in view of the ease of application to the skin, it is required that the desired viscosity of the cosmetic remains stable for a long period.

The present invention has been made in view of the aforementioned existing circumstances. An object of the present invention is to provide an emulsion-type base makeup cosmetic which has good viscosity stability, which can provide a natural skin color finish independent of a light source, which is superior in terms of the use sensation, and which suppresses staining of skin caused by the bleeding out of a colorant component; and to provide a method of production thereof.

### Solution to Problem

The present invention is defined in the appended claims and is as follows.
[1] A emulsion-type base makeup cosmetic which includes the following components (a), (b), (c), and (d):
   (a) a powdered pigment comprising a red composite pigment in which lithol rubine BCA is intercalated into an inorganic substance,
   (b) powdered pigments comprising an extender pigment, a coloring pigment, and a pearl pigment, and which are different from the component (a),
   (c) an oily material, and
   (d) a water-soluble calcium salt which is at least one selected from calcium chloride, calcium lactate, or calcium acetate.
[2] The emulsion-type base makeup cosmetic according to [1], wherein a content of the component (d) is in an amount of from 0.1% by mass to 5% by mass with respect to a total mass of the base makeup cosmetic.
[3] The emulsion-type base makeup cosmetic according to [1] or [2], wherein a reflectance spectrum of an apparent color of the base makeup cosmetic has a local minimal value in a wavelength region of from 500 nm to 600 nm.
[4] The emulsion-type base makeup cosmetic according to any one of [1] to [3], wherein the pearl pigment is included in an amount of from 5% by mass to 30% by mass with respect to a total mass of the component (b).
[5] A method of producing an emulsion-type base makeup cosmetic, the method comprising preparing an oil phase wherein the oil phase comprises (a) a powdered pigment comprising a red composite pigment in which lithol rubine BCA is intercalated into an inorganic substance; (b) powdered pigments comprising an extender pigment, a coloring pigment, and a pearl pigment, and which are different from the component (a); and (c) an oily material, and mixing the oil phase with an aqueous phase that comprises (d) a water-soluble calcium salt which is at least one selected from calcium chloride, calcium lactate, or calcium acetate, to form an emulsion.
[6] The method of producing an emulsion-type base makeup cosmetic according to [5], wherein a content of the component (d) is in an amount of from 0.1% by mass to 5% by mass with respect to a total mass of the base makeup cosmetic.

### Advantageous Effects of Invention

According to the present invention, an emulsion-type base makeup cosmetic, which has good viscosity stability, which can provide a natural skin color finish independent of a light source, which is superior in terms of use sensation, and which is suppresses a staining of skin caused by the bleeding-out of a colorant component, as well as a method of production thereof, can be provided.

### BRIEF DESCRIPTION OF DRAWINGS

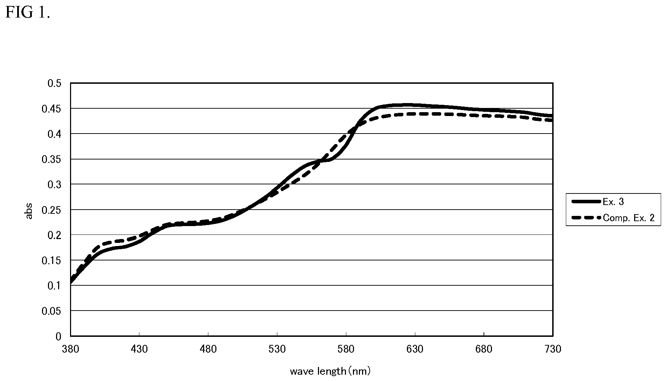

Figure 1 is a graph showing the reflectance spectra of the apparent color of the liquid foundations obtained in each of Example 3 and Comparative Example 2.

### DESCRIPTION OF EMBODIMENTS

The base makeup cosmetic and the method of production thereof according to the present invention will be described below in detail.

In the present specification, a numerical range expressed using "to" means a range including the numerical values before and after "to" as the minimum and maximum values, respectively.

In a case in which the amount of a component in the composition is indicated in the invention, when there are plural substances corresponding to the component in the composition, the indicated amount means the total amount of the plural substances present in the composition, unless specifically stated otherwise.

In the present specification, the term "process" encompasses an independent process, as well as a process that cannot be clearly distinguished from another process but yet achieves the expected effect of the process of interest.

### (1) Emulsion-type base makeup cosmetic

The emulsion-type base makeup cosmetic of the present invention (hereinafter sometimes referred to as "cosmetic ") includes the following components (a), (b), (c), and (d):
(a) a red powdered pigment comprising a red composite pigment in which lithol rubine BCA is intercalated into an inorganic substance,
(b) powdered pigments including an extender pigment, a coloring pigment, and a pearl pigment, and which are different from the component (a),
(c) an oily material, and
(d) a water-soluble calcium salt which is at least one selected from calcium chloride, calcium lactate, or calcium acetate.

The cosmetic of the present invention has good viscosity stability, can provide a natural skin color finish independent of a light source (alleviating yellow dulling), is superior in terms of the use sensation, and can efficiently suppress uneven coloring on a cosmetic surface or staining of skin caused by dissolution of a colorant component.

The cosmetic of the present invention includes an emulsion obtained by mixing an oil phase and an aqueous phase and then emulsifying the resultant. The essential and optional components included in the cosmetic of the present invention will be described below.

### <Component (a)>

The cosmetic of the present invention includes, as the component (a), a powdered pigment which comprises a red composite pigment in which lithol rubine BCA is intercalated into an inorganic substance.

The component (a) can function as a colorant component in the cosmetic of the present invention, and can effectively contribute to give a natural skin color finish independent of a light source (alleviating yellow dulling).

The powdered pigment included in the cosmetic of the present invention as the component (a) is a composite pigment which includes lithol rubine BCA as an essential element thereof.

The lithol rubine BCA is a red monoazo pigment known as Pigment Red 202.

The component (a) is a red composite pigment having a structure in which lithol rubine BCA is intercalated into an inorganic substance (hereinafter sometimes referred to as "specific red composite pigment"). Inclusion of the specific red composite pigment provides better suppression of dissolution of the colorant component.

Here, intercalation or to be intercalated means a phenomenon in which a molecule, an atom or an ion is inserted between layers of a substance having a layered structure.

More specifically, the specific red composite pigment powder in the present invention is obtained by forming a composite of lithol rubine BCA with a planar layered inorganic powder and two or more kinds of inorganic hydroxides thereby fixing.

The specific red composite pigment can be obtained by once completely dissolving lithol rubine BCA in water, separating two kinds of inorganic hydroxides out in the presence of two kinds of inorganic salts while adjusting the pH, adding a planar layered inorganic powder, and forming a composite of the organic dye stuff, the two kinds of inorganic hydroxides, and the planar layered inorganic powder, thereby chemically fixing. In particular, the production can be made according to the method of production described in JP-A No. 2003-105225.

The content of pure dye stuff component in the specific red composite pigment powder is preferably 5 % by mass or more but less than 50 % by mass, and more preferably from 10 % by mass to 30 % by mass, from viewpoints of color development and suppression of a dye stuff component into a solvent.

Examples of the inorganic salt include a metallic chloride, such as magnesium chloride, and aluminum chloride. Examples of the inorganic hydroxide include magnesium hydroxide and aluminum hydroxide. Examples of the planar layered inorganic powder include montmorillonite, beidellite, hectorite, saponite, nontronite, or an expansive fluorine-bearing mica.

As the specific red composite pigment, a commercially available product may be used and examples thereof include HNB RED7 (trade name, composite pigments including lithol rubine BCA, manufactured by Daito Kasei Kogyo Co., Ltd.), and HNB RED6 trade name, composite pigments including lithol rubine BCA, manufactured by Daito Kasei Kogyo Co., Ltd.)

As for the particle diameter of the powdered pigment included as the component (A), the average primary particle diameter is preferably from 1 µm to 10 µm, and more preferably from 2 µm to 8 µm.

The content of the powdered pigment included as the component (A) in the cosmetic of the present invention is preferably 10 % by mass or more with respect to a total mass of red pigment required to adjust the color of a cosmetic to a targeted color, and more preferably 50 % by mass or more, more preferably 75 % by mass or more, and further preferably 100 % by mass.

The content of the specific red composite pigment is adjusted within the above range considering the color forming efficiency of a relevant type.

The total content of the powdered pigment included as the component (a) in the cosmetic of the present invention is preferably from 0.01% by mass to 5% by mass, more preferably from 0.03% by mass to 4% by mass, and still more preferably from 0.05% by mass to 3% by mass, with respect to a total mass of the cosmetic.

The component (a) contained in the cosmetic of the present invention may be a single kind of pigment or a combination of two or more kinds thereof. For example, the component (a) may be only a composite pigment which includes lithol rubine BCA as an essential element (the specific red composite pigment) or a combination of a red composite pigment in which lithol rubine BCA is intercalated into an inorganic substance and lithol rubine BCA.

### <Component (b)>

The cosmetic of the present invention includes, as the component (b), powdered pigments which include an extender pigment, a coloring pigment, and a pearl pigment. The powdered pigments contained as the component (b) are a powdered pigments which are different from the component (a).

The total content of the powdered pigment contained as the component (b) in the cosmetic of the present invention is preferably from 1% by mass to 50% by mass, more preferably from 5% by mass to 40% by mass, and still more preferably from 10% by mass to 30% by mass, with respect to a total mass of the cosmetic.

### <<Extender pigment>>

The extender pigment means a pigment that does not substantially contribute to adjustment of a hue.

Examples of the extender pigment include mica, synthetic phlogopite, talc, kaolin, mica, sericite, manganese carbonate, calcium carbonate, silicic anhydride, aluminum oxide, barium sulfate, or the like.

As the extender pigment, a commercially available product may also be used, and examples thereof include SERICITE FSE (manufactured by Sanshin Mining Ind. Co., Ltd., trade name), TALK JA-46R (manufactured by Asada Milling Co., Ltd., trade name), a synthetic phlogopite PDM series (manufactured by Topy Industries Ltd., trade name), and OTS-2 SERICITE FSE and OTS-2 TALK JA-46R (All of them are manufactured by Daito Kasei Kogyo Co., Ltd.).

As for the particle diameter of the extender pigment, its mean primary particle size is preferably from 1 µm to 100 µm, and more preferably from 5 µm to 80 µm.

The particle size of a pigment in the present invention can be measured by preparing a dispersion in a solvent containing the pigment which is a measurement object at a given concentration, and measuring the same by any of various commercial measurement instruments based on a principle of laser light scattering (for example, a laser diffraction scattering particle size distribution analyzer LMS-30 (manufactured by Seishin Enterprise Co., Ltd., trade name)).

The extender pigment is preferably contained in an amount of from 5% by mass to 40% by mass with respect to a total mass of the cosmetic.

The cosmetic of the present invention may contain one kind of extender pigment singly, or two or more kinds thereof.

### <<Coloring pigment>>

A coloring pigment is further contained as a colorant component other than the component (a). The coloring pigment means a pigment that contributes to adjustment of a hue and that is not a pearl pigment.

Examples of the coloring pigment include an iron oxide such as yellow iron oxide, red iron oxide, and black iron oxide, or a white pigment.

Among the coloring pigments, from viewpoints of color tone adjustment and suppression of dissolution into a solvent (bleeding-out), an iron oxide is preferable, and red iron oxide is more preferable.

In a case titanium oxide, zinc oxide or the like is contained as a white pigment, the white pigment may also have a function as a masking agent against blemishes, freckles or the like, or an ultraviolet ray protecting agent.

With respect to the shape and the particle diameter of the coloring pigments, for example, with respect to the shape and the particle diameter of a white pigment, a white pigment which is spherical and from several nm to several hundreds nm is preferably used, and with respect to the shape and the particle diameter of an iron oxide, an iron oxide which is spherical or acicular and from several nm to several hundreds nm is preferably used.

As the coloring pigment, a commercially available product may be used, and examples thereof include OTS-2 TiO2 CR-50, OTS-2 YELLOW LLXLO, OTS-2 RED R-516L, and OTS-2 BLACK BL-100 (All of them are manufactured by Daito Kasei Kogyo Co., Ltd.).

In the present invention, according to need, a composite pigment in which a pigment other than lithol rubine BCA is intercalated into an inorganic substance may be used as the coloring pigment, to the extent advantageous effects of the present invention are not impaired.

The addition amount of the coloring pigment is adjusted appropriately so as to attain the color tone of the entire cosmetic to the target tone.

The cosmetic of the present invention may contain one kind of coloring pigment singly, or two or more kinds thereof.

### <<Pearl pigment>>

The pearl pigment means a pigment that contributes to adjustment of a hue and that has a pearly luster

Examples of the pearl pigment include titanium oxide-coated mica (titanated mica), titanium oxide-coated glass flake, titanium oxide-coated talc, or the like. A pearl pigment in which the titanium oxide coating has a multiple layer structure, or a multiple layer structure including a silicon oxide layer, may also be preferably used.

As for the particle diameter of the pearl pigment, its mean primary particle diameter is preferably from 0.5 µm to 100 µm, and more preferably from 1 µm to 80 µm.

The content of the pearl pigment with respect to a total mass of the cosmetic is preferably 0.5% by mass to 30% by mass, more preferably from 1% by mass to 20% by mass, and still more preferably from 2% by mass to 15% by mass.

In view of optical properties, the pearl pigment is more preferably included in an amount of from 5% by mass to 30% by mass and still more preferably from 10% by mass to 20% by mass, with respect to a total mass of the powdered pigments included as the component (b).

The pearl pigment contained in the cosmetic of the present invention may be one kind of pearl pigment singly, or two or more kinds thereof. The pearl pigment is preferably added in such a ratio that the hue angle of the reflectance color of the entire pearl pigment contained in the cosmetic is within a range from 40° to 80°.

As a pearl pigment, a commercially available product may be used, and examples thereof include RONAFLAIR BALANCE GOLD, TRANS PRISMA RED, TIMIRON SUPER SILK MP-1005 (All of them are manufactured by MERCK), and FLAMENCO series (manufactured by BASF).

As the pearl pigment, a gold pearl pigment and a red pearl pigment are preferable, and a mixture of these pearl pigments is also preferable.

Examples of a preferable combination of the powdered pigments contained as the component (B) in the cosmetic of the present intervention include a combination of at least one kind of extender pigment selected from mica, sericite, or talc, at least one kind of coloring pigment selected from yellow iron oxide, red iron oxide, black iron oxide, or titanium oxide, and at least one kind of pearl pigment selected from a gold pearl pigment or a red pearl pigment.

### <Component (c)>

The cosmetic of the present invention includes an oily material as the component (c).

Examples of the oily material include oils commonly used in a cosmetic, such as a silicone oil, a liquid paraffin, a petroleum jelly, a paraffin wax, a squalane, a beeswax, carnauba wax, an olive oil, lanolins, a higher alcohol, a fatty acid, a higher fatty acid, an ester oil, ceresin, a microcrystalline wax, candelilla wax, a diglyceride, a triglyceride, a perfluoropolyether, a perfluorodecalin, a perfluorooctane, jojoba oil, octyldodecyl myristate, or neopentyl glycol diethylhexanoate.

The content of the oily material contained as the component (c) in the cosmetic of the present invention is preferably from 5% by mass to 60% by mass, more preferably from 10% by mass to 50% by mass, and still more preferably from 15% by mass to 40% by mass, with respect to a total mass of the cosmetic.

The oily material included as the component (c) in the cosmetic of the present invention may be one kind of the oily material or a combination of two or more kinds thereof.

### <Component (d)>

The cosmetic of the present invention includes a water-soluble calcium salt as the component (d).

The water-soluble calcium salt is contained as a solution dissolving in an aqueous medium such as water that is included in an aqueous phase in the cosmetic of the present invention.

Here, the water-soluble calcium salt means a calcium salt which is soluble to the extent of at least 3% by mass in distilled water at 25°C.

In the cosmetic of the present invention, the water-soluble calcium salt as the component (d) contributes to favorable viscosity stability and can suppress dissolution of the colorant component without impairing the use sensation.

Although it is necessary for the desired viscosity of an emulsion-type base makeup cosmetic to remain stable for a long period, conventional cosmetics may exhibit reduced viscosity. Simple addition of a compound that acts as a viscosity enhancer would be sufficient to improve the viscosity stability of the cosmetic. The addition of a viscosity enhancer, however, may result in a cosmetic having an unpleasant use sensation such as tackiness. In addition, the addition of a viscosity enhancer cannot suppress dissolution of the colorant component.

In contrast, the cosmetic of the present invention includes the water-soluble calcium salt as the component (d), and thus specifically has the beneficial effects of exhibiting good viscosity stability without impairing the use sensation and suppressing dissolution of the colorant component. On the other hand, a water-soluble inorganic salt other than the water-soluble calcium salt cannot provide such effects.

The water-soluble calcium salt is at least one selected from calcium chloride, calcium lactate, or calcium acetate. Among these water-soluble calcium salts, calcium chloride is more preferable in terms of ease of preparation and usability.

The content of the water-soluble calcium salt contained as the component (d) in the cosmetic of the present invention is preferably from 0.1% by mass to 5% by mass, and more preferably from 0.5% by mass to 3% by mass, with respect to a total mass of the cosmetic.

The content of the water-soluble calcium salt contained as the component (d) is preferably from 0.1% by mass to 10% by mass, more preferably from 0.1% by mass to 5% by mass, and still more preferably from 0.1% by mass to 3% by mass, with respect to a total mass of all the pigment components contained in the cosmetic (total mass of the components (a) and (b)).

### <Other additives>

The cosmetic of the present invention may contain, according to need, a component usually added to a cosmetic, such as a surfactant, a water-soluble polymer, a humectant, an antiseptic agent, a medicinal agent, an ultraviolet absorber, a dye stuff, an inorganic salt or an organic salt, a fragrance, a chelating reagent, a pH adjuster, water, or the like, to the extent advantageous effects of the present invention are not impaired.

Examples of the surfactant include surfactants commonly used in a cosmetic involving a nonionic surfactant, such as a polyoxyethylene alkyl ether, a polyoxyethylene fatty acid ester, a polyoxyethylene sorbitan fatty acid ester, a glycerol fatty acid ester, a polyglycerol fatty acid ester, a polyoxyethylene glycerol fatty acid ester, a polyoxyethylene hydrogenated castor oil, or a polyoxyethylene sorbitol fatty acid ester; an anionic surfactant as represented by a fatty acid soap, such as sodium stearate, or triethanolamine palmitate; a cationic surfactant; or an amphoteric surfactant.

Examples of the water-soluble polymer include water-soluble polymers commonly used in a cosmetic, such as carboxymethyl cellulose, methyl cellulose, hydroxymethyl cellulose, polyvinyl alcohol, polyvinylpyrrolidone, tragacanth gum, carrageenan, locust bean gum, dextrin, a dextrin fatty acid ester, a carboxyvinyl polymer, xanthan gum, gelatin, sodium alginate, gum arabic, or water-soluble collagen.

Examples of the humectant include humectants commonly used in a cosmetic, such as sorbitol, xylitol, glycerol, maltitol, propylene glycol, 1,3-butylene glycol, 1,4-butylene glycol, sodium pyrrolidone carboxylate, lactic acid, sodium lactate, or polyethylene glycol.

Examples of the antiseptic agent include antiseptic agents commonly used in a cosmetic, such as a *p*-oxybenzoic acid alkyl ester, sodium benzoate, potassium sorbate, or phenoxyethanol.

Examples of the medicinal agent include medicinal agents commonly used in a cosmetic, such as vitamins, a herbal medicine, an anti-inflammatory agent, or an antimicrobial agent.

Examples of the ultraviolet absorber include ultraviolet absorbers commonly used in a cosmetic, such as a *p*-aminobenzoic acid type ultraviolet absorber, an anthranil type ultraviolet absorber, a salicylic acid type ultraviolet absorber, a cinnamic acid type ultraviolet absorber, or a benzophenone type ultraviolet absorber.

Examples of the dye stuff include a dye stuff applicable to a cosmetic such as a natural dye stuff, examples thereof including a *Haematococcus* algae dye stuff, carminic acid, laccaic acid, brazilin, or crocin.

The cosmetic of the present invention preferably exhibits a reflectance spectrum of the apparent color thereof having a local minimal value in a wavelength region from 500 nm to 600 nm from a viewpoint of suppression of yellow dulling.

The reflectance spectrum herein is a value obtained by measurement by SPECTROLINO (trade name) (manufactured by Gretag Machbeth).

The cosmetic of the present invention can be suitably produced by a method of producing an emulsion-type base makeup cosmetic (a production method of the present invention) as described below in detail.

### (2) Method of producing emulsion-type base makeup cosmetic

The method of producing an emulsion-type base makeup cosmetic of the present invention includes preparing an oil phase including (a) a powdered pigment comprising a red composite pigment in which lithol rubine BCA is intercalated into an inorganic substance, (b) powdered pigments including an extender pigment, a coloring pigment, and a pearl pigment and which are different from the component (a), and (c) an oily material; and mixing the oil phase with an aqueous phase that includes (d) a water-soluble calcium salt which is at least one selected from calcium chloride, calcium lactate, or calcium acetate, to form an emulsion.

Each of the essential components of (a) the powdered pigment comprising a red composite pigment in which lithol rubine BCA is intercalated into an inorganic substance (component (a)), (b) the powdered pigments including an extender pigment, a coloring pigment, and a pearl pigment and which are different from the component (a) (component (b)), (c) the oily material (component (c)), and (d) the water-soluble calcium salt which is at least one selected from calcium chloride, calcium lactate, or calcium acetate (component (d)) used in the present production method, and other additives which are used as desired, are as described above for the components (a) to (d) and the other additives of the cosmetic of the present invention. The preferred embodiments are also as described above for the cosmetic of the present invention.

The oil phase is prepared by mixing the powdered pigments (components (a) and (b)), the oily material (component (c)), and an optional additive as an oil phase component.

The aqueous phase is prepared by dissolving the water-soluble calcium salt (component (d)) and an optional additive as an aqueous phase component into an aqueous medium such as water.

Any conventionally known emulsification techniques can be used in the production method of the present invention without limitation. An example of the emulsification techniques which can be used in the present invention includes, for example, a technique of separately heating the aqueous phase and the oil phase to about 80°C, slowly mixing the phases, emulsifying the resultant mixture in an emulsification apparatus to form an emulsion, and allowing the emulsion to cool to room temperature.

The resultant emulsion preferably has a ratio of the oil phase (% by mass)/the aqueous phase (% by mass) of from 90/10 to 30/70, more preferably from 85/15 to 40/60, and more preferably from 80/20 to 50/50, although the ratio is not limited thereto.

The cosmetic of the present invention may be, for example, a liquid foundation, a cosmetic primer, a concealer, and a color correcting base.

### EXAMPLES

The present invention is described below by way of Examples in details, provided that the present invention be not limited thereto by any means. The "part" herein is by mass unless otherwise specified.

[Examples 2 to 6 and 8 to 10, Reference Examples 1 and 7, and Comparative Examples 1 to 12]

### (1) Preparation of Liquid Foundations

Components of A, B, and C phases described in Tables 1 and 2 below were individually weighed out.

The B phase was added to a homogenizer chamber, and the A phase was added in small portions with stirring. Then the temperature in the chamber was raised to 80°C, and the chamber was stirred to completely dissolve the components of the B phase. Another homogenizer chamber containing the C phase was warmed to 80°C and then stirred to completely dissolve the components. Then, the mixture of the B and A phases and the aqueous solution of the C phase were cooled to 50°C. The C phase was added in small portions to the B phase in the homogenizer under stirring. The resultant mixture was sufficiently emulsified with stirring to provide a respective liquid foundation of Examples 2 to 6 and 8 to 10, Reference Examples 1 and 7, and Comparative Examples 1 to 12.

**[Table 1] In Table 1 Example 1 is a Reference Example.**

| (% by mass ) | | | | | | | | | | | | | | |
|---|---|---|---|---|---|---|---|---|---|---|---|---|---|---|
| Component | | Comparative Example 1 | Comparative Example 2 | Comparative Example 3 | Comparative Example 4 | Comparative Example 5 | Comparative Example 6 | Comparative Example 7 | Example 1 | Example 2 | Example 3 | Example 4 | Example 5 | Example 6 |
| A Phase | Pigment Red 202*¹ [Component (a)] | 0.1 | | | | | | | 0.1 | | | | | |
| | Specific red composite pigment 1*² [Component (a)] | | 1.0 | 1.0 | 1.0 | 1.0 | 1.0 | | | 1.0 | 1.0 | 1.0 | 1.0 | 1.0 |
| | Extender pigment*³ [Component (b)] | 15.0 | 15.0 | 15.0 | 15.0 | 15.0 | 15.0 | 15.0 | 15.0 | 15.0 | 15.0 | 15.0 | 15.0 | 15.0 |
| | Coloring pigment*⁴ [Component (b)] | 1.0 | 1.0 | 1.0 | 1.0 | 1.0 | 1.0 | 1.3 | 1.0 | 1.0 | 1.0 | 1.0 | 1.0 | 1.0 |
| | Pearl pigment*⁵ [Component (b)] | 3.0 | 30 | 3.0 | 3.0 | 3.0 | 3.0 | 3.0 | 3.0 | 3.0 | 3.0 | 3.0 | 3.0 | 3.0 |
| B Phase | Cyclomethicone [Component (c)] | 25.0 | 25.0 | 25.0 | 25.0 | 25.0 | 25.0 | 25.0 | 25.0 | 25.0 | 25.0 | 25.0 | 25.0 | 25.0 |
| | Dimethicone Copolyol [Component (c)] | 5.0 | 5.0 | 5.0 | 5.0 | 5.0 | 5.0 | 5.0 | 5.0 | 5.0 | 5.0 | 5.0 | 5.0 | 5.0 |
| | Lauryl PEG-9 Polydimethylsiloxyethyl Dimethicone [Component (C)] | 3.0 | 3.0 | 3.0 | 3.0 | 3.0 | 3.0 | 3.0 | 3.0 | 3.0 | 3.0 | 3.0 | 3.0 | 3.0 |
| | Squalane [Component (C)] | 0.1 | 0.1 | 0.1 | 0.1 | 0.1 | 0.1 | 0.1 | 0.1 | 0.1 | 0.1 | 0.1 | 0.1 | 0.1 |
| | Sorbitan Sesquiisostearate | 1.0 | 1.0 | 1.0 | 1.0 | 1.0 | 1.0 | 1.0 | 1.0 | 1.0 | 1.0 | 1.0 | 1.0 | 1.0 |
| | Disteardimonium Hectorite | 0.8 | 0.8 | 0.8 | 0.8 | 0.8 | 0.8 | 0.8 | 0.8 | 0.8 | 0.8 | 0.8 | 0.8 | 0.8 |
| | Ethylhexyl Methoxycinnamate | 2.5 | 2.5 | 2.5 | 2.5 | 2.5 | 2.5 | 2.5 | 2.5 | 2.5 | 2.5 | 2.5 | 2.5 | 2.5 |
| | Haematococcus Pluvialis Oil | 0.1 | 0.1 | 0.1 | 0.1 | 0.1 | 0.1 | 0.1 | 0.1 | 0.1 | 0.1 | 0.1 | 0.1 | 0.1 |
| | Tocopherol | 0.1 | 0.1 | 0.1 | 0.1 | 0.1 | 0.1 | 0.1 | 0.1 | 0.1 | 0.1 | 0.1 | 0.1 | 0.1 |
| | Rosa Damascena Flower Oil | 0.1 | 0.1 | 0.1 | 0.1 | 0.1 | 0.1 | 0.1 | 0.1 | 0.1 | 0.1 | 0.1 | 0.1 | 0.1 |
| | Fragrance | 0.1 | 0.1 | 0.1 | 0.1 | 0.1 | 0.1 | 0.1 | 0.1 | 0.1 | 0.1 | 0.1 | 0.1 | 0.1 |
| | Phenoxyethanol | 0.3 | 0.3 | 0.3 | 0.3 | 0.3 | 0.3 | 0.3 | 0.3 | 0.3 | 0.3 | 0.3 | 0.3 | 0.3 |
| C Phase | Glycerin | 10.0 | 10.0 | 10.0 | 10.0 | 10.0 | 10.0 | 10.0 | 10.0 | 10.0 | 10.0 | 10.0 | 10.0 | 10.0 |
| | Dipropylene Glycol | 4.0 | 4.0 | 4.0 | 4.0 | 4.0 | 4.0 | 4.0 | 4.0 | 4.0 | 4.0 | 4.0 | 4.0 | 4.0 |
| | 1.3-Butylene Glycol | 3.0 | 3.0 | 3.0 | 3.0 | 3.0 | 3.0 | 3.0 | 3.0 | 3.0 | 3.0 | 3.0 | 3.0 | 3.0 |
| | Water-Soluble Collagen | 0.1 | 0.1 | 0.1 | 0.1 | 0.1 | 0.1 | 0.1 | 0.1 | 0.1 | 0.1 | 0.1 | 0.1 | 0.1 |
| | Royal Jelly Extract | 0.1 | 0.1 | 0.1 | 0.1 | 0.1 | 0.1 | 0.1 | 0.1 | 0.1 | 0.1 | 0.1 | 0.1 | 0.1 |
| | Xanthan Gum | 1.0 | 1.0 | | | | | | | | | | | |
| | Hydroxyethyl Cellulose | | | 1.0 | | | | | | | | | | |
| | Sodium Citrate | | | | 1.0 | | | | | | | | | |
| | Magnesium Chloride | | | | | 1.0 | | | | | | | | |
| | Aluminum Chloride | | | | | | 1.0 | | | | | | | |
| | Calcium Chloride [Component (d)] | | | | | | | 1.0 | 1.0 | 2.0 | 1.0 | 0.5 | | |
| | Calcium Lactate [Component (d)] | | | | | | | | | | | | 1.0 | |
| | Calcium Acetate [Component (d)] | | | | | | | | | | | | | 1.0 |
| | Ion-Exchanged Water | Balance | Balance | Balance | Balance | Balance | Balance | Balance | Balance | Balance | Balance | Balance | Balance | Balance |
| Content of Pearl pigment Based on Total Mass of Component (b)(%) | | 15.8 | 15.8 | 15.8 | 15.8 | 15.8 | 15.8 | 15.5 | 15.8 | 15.8 | 15.8 | 15.8 | 15.8 | 15.8 |
| Evaluation | Alleviating yellow dulling | A | B | B | A | A | A | C | A | A | A | A | A | A |
| | Dissolution property | C | B | B | C | C | C | A | A | A | A | A | A | A |
| | Staining of skin | C | B | B | C | C | C | A | A | A | A | A | A | A |
| | Viscosity stability | B | B | B | C | C | C | A | S | S | S | S | A | A |
| | Use sensation | C | C | C | A | B | B | A | A | A | A | A | A | A |

| | | | | | | | | | | | | | | |
|---|---|---|---|---|---|---|---|---|---|---|---|---|---|---|
| *1: Pigment Red 202 from Kishi Kasei Co., Ltd. *2: HNB RED 7 from Daito Kasei Kogvo Co., Ltd. *3: Mixture of OTS-2 SERICITE FSE and OTS-2 TALK JA-46R from Daito Kasei Kogvo Co., Ltd. in a ratio of 7:3 *4: Mixture of OTS-2 TiO2 CR-50. OTS-2 YELLOW LLXLO. OTS-2 RE DR-516L and OTS-2 BLACK BL-100 from Daito Kasei Kogvo Co., Ltd. in a ratio of 78:19:1:2 *5: Mixture of RONAFLAIR BALANCE GOLD and TRANS PRISMA RED from MERCK & Co. in a ratio of 7:3 | | | | | | | | | | | | | | |

**[Table 2] In Table 2 Example 7 is a Reference Example.**

| (% by mass) | | | | | | | | | | |
|---|---|---|---|---|---|---|---|---|---|---|
| Component | | Comparative Example 8 | Comparative Example 9 | Comparative Example 10 | Comparative Example 11 | Comparative Example 12 | Example 7 | Example 8 | Example 9 | Example 10 |
| A Phase | Pigment Red 202*¹ [Component (a)] | 0.1 | | | | | 0.1 | | | |
| | Specific red composite pigment 1*² [Component (a)] | | 1.0 | 1.0 | 1.0 | | | 1.0 | 1.0 | 1.0 |
| | Extender pigment*³ [Component (b)] | 15.0 | 15.0 | 15.0 | 15.0 | 15.0 | 15.0 | 15.0 | 15.0 | 15.0 |
| | Coloring pigment*⁴ [Component (b)] | 1.0 | 1.0 | 1.0 | 1.0 | 1.3 | 1.0 | 1.0 | 1.0 | 1.0 |
| | Pearl pigment*⁵ [Component (b)] | 3.0 | 3.0 | 3.0 | 3.0 | 3.0 | 3.0 | 3.0 | 3.0 | 3.0 |
| B Phase | Cyclomethicone [Component (c)] | 24.0 | 24.0 | 24.0 | 24.0 | 24.0 | 24.0 | 24.0 | 24.0 | 24.0 |
| | PEG-10 Dimethicone, Dimethicone [Component (c)] | 5.0 | 5.0 | 5.0 | 5.0 | 5.0 | 5.0 | 5.0 | 5.0 | 5.0 |
| | Lauryl PEG-9 Polydimethylsiloxyethyl Dimethicone [Component (C)] | 3.0 | 3.0 | 3.0 | 3.0 | 3.0 | 3.0 | 3.0 | 3.0 | 3.0 |
| | Squalane [Component (C)] | 2.0 | 2.0 | 2.0 | 2.0 | 2.0 | 2.0 | 2.0 | 2.0 | 2.0 |
| | Isotridecyl Isononanoate | 5.0 | 5.0 | 5.0 | 5.0 | 5.0 | 5.0 | 5.0 | 5.0 | 5.0 |
| | Octyldodecyl/Phytosteryl/Behenyl Lauroyl Glutamate | 1.5 | 1.5 | 1.5 | 1.5 | 1.5 | 1.5 | 1.5 | 1.5 | 1.5 |
| | Polyglyceryl-2 Diisostearate | 2.0 | 2.0 | 2.0 | 2.0 | 2.0 | 2.0 | 2.0 | 2.0 | 2.0 |
| | Ethylhexyl Methoxycinnamate | 2.5 | 2.5 | 2.5 | 2.5 | 2.5 | 2.5 | 2.5 | 2.5 | 2.5 |
| | Haematococcus Pluvialis Oil | 0.1 | 0.1 | 0.1 | 0.1 | 0.1 | 0.1 | 0.1 | 0.1 | 0.1 |
| | Tocopherol | 0.1 | 0.1 | 0.1 | 0.1 | 0.1 | 0.1 | 0.1 | 0.1 | 0.1 |
| | Rosa Damascena Flower Oil | 0.1 | 0.1 | 0.1 | 0.1 | 0.1 | 0.1 | 0.1 | 0.1 | 0.1 |
| | Fragrance | 0.1 | 0.1 | 0.1 | 0.1 | 0.1 | 0.1 | 0.1 | 0.1 | 0.1 |
| | Phenoxyethanol | 0.3 | 0.3 | 0.3 | 0.3 | 0.3 | 0.3 | 0.3 | 0.3 | 0.3 |
| C Phase | 1,3-Butylene Glycol | 5.0 | 5.0 | 5.0 | 5.0 | 5.0 | 5.0 | 5.0 | 5.0 | 5.0 |
| | Water-Soluble Collagen | 0.1 | 0.1 | 0.1 | 0.1 | 0.1 | 0.1 | 0.1 | 0.1 | 0.1 |
| | Royal Jelly Extract | 0.1 | 0.1 | 0.1 | 0.1 | 0.1 | 0.1 | 0.1 | 0.1 | 0.1 |
| | Xanthan Gum | | | 1.0 | | | | | | |
| | Sodium Citrate | 1.0 | 1.0 | | | | | | | |
| | Magnesium Chloride | | | | 1.0 | | | | | |
| | Calcium Chloride [Component (d)] | | | | | 1.0 | 1.0 | 0.5 | | |
| | Calcium Lactate [Component (d)] | | | | | | | | 1.0 | |
| | Calcium Acetate [Component (d)] | | | | | | | | | 1.0 |
| | Ion-Exchanged Water | Balance | Balance | Balance | Balance | Balance | Balance | Balance | Balance | Balance |
| Content of Pearl pigment Based on Total Mass of Component (b) (%) | | 15.8 | 15.8 | 15.8 | 15.8 | 15.8 | 15.5 | 15.8 | 15.8 | 15.8 |
| Evaluation | Alleviating yellow dulling | A | A | B | A | C | A | A | A | A |
| | Dissolution property | C | C | B | C | A | A | A | A | A |
| | Staining of skin | C | C | B | C | A | A | A | A | A |
| | Viscosity stability | C | C | B | C | A | S | S | A | A |
| | Use sensation | A | A | C | B | A | A | A | A | A |

| | | | | | | | | | | |
|---|---|---|---|---|---|---|---|---|---|---|
| *1: Pigment Red 202 from Kishi Kasei Co., Ltd. *2: HNB RED 7 from Daito Kasei Kogvo Co., Ltd. *3: Mixture of OTS-2 SERICITE FSE and OTS-2 TALK JA-46R from Daito Kasei Kogyo Co., Ltd. in a ratio of 7:3 *4: Mixture of OTS-2 TiO, CR-50. OTS-2 YELLOW LLXLO. OTS-2 RE DR-516L. and OTS-2 BLACK BL-100 from Daito Kasei Kogvo Co., Ltd. in a ratio of 78:19:1:2 *5: Mixture of RONAFLAIR BALANCE GOLD and TRANS PRISMA RED from MERCK & Co. in a ratio of 7:3 | | | | | | | | | | |

### (2) Evaluation of foundations

### [Alleviating yellow dulling]

Each of the liquid foundations obtained in Examples 2 to 6 and 8 to 10, Reference Examples 1 and 7, and Comparative Examples 1 to 12 was applied by a puff to the face of the same female test subject, and the color tone of the skin color after the application was visually observed under a three wavelength fluorescent light with the color temperature of 5000K. Then the female test subject was moved to a place in direct sunlight (midday sunlight under a clear sky, 5000K), and the relevant color tone change of the skin color was visually observed and evaluation was performed according to the following evaluation criteria. Good rating in this evaluation means that a natural skin color finish can be obtained independent of a light source. The results are shown in Tables 1 and 2.
A: Changed to red tone (no yellow dulling)
B: No change or slight change to yellow tone
C: Changed to yellowish tone

### [Dissolution property (Color unevenness)]

Each of the liquid foundations obtained in Examples 2 to 6 and 8 to 10, Reference Examples 1 and 7, and Comparative Examples 1 to 12 was filled in a transparent container, and the existence of unevenness of red tone was visually observed and evaluated according to the following evaluation criteria. The results are shown in Tables 1 and 2.

### - Evaluation Criteria -

A: Absolutely no unevenness of red tone
B: Only slight unevenness of red tone recognized
C: Remarkable unevenness of red tone recognized

### [Staining of skin]

After 6 hours from putting each of the obtained liquid foundations of Examples 2 to 6 and 8 to 10, Reference Examples 1 and 7, and Comparative Examples 1 to 12 on the skin, the foundation was rinsed off with water without using a facial cleanser. Thereafter the degree of staining of a red pigment on the skin was visually observed and evaluated according to the following evaluation criteria. The results are shown in Tables 1 and 2.

### -Evaluation criteria-

A: Absolutely no red staining recognized
B: Slight red staining recognized
C: Red staining was recognized easily visually

### [Viscosity stability (Dispersion stability)]

Each of the liquid foundations obtained in Examples 2 to 6 and 8 to 10, Reference Examples 1 and 7, and Comparative Examples 1 to 12 was placed in a constant temperature bath at 50°C for 4 weeks. On day 1 and day 28, the viscosity of the foundations was measured, and the variation range of viscosity (viscosity difference) was evaluated according to the following evaluation criteria. The results are shown in Tables 1 and 2.

### - Evaluation Criteria -

S: Viscosity difference is less than 3000 mPa·s.
A: Viscosity difference is 3000 mPa·s or more and less than 5000 mPa·s.
B: Viscosity difference is 5000 mPa·s or more and less than 10000 mPa·s.
C: Viscosity difference is 10000 mPa·s or more.

### [Use sensation (Tackiness and Moistness)]

With respect to each of the obtained liquid foundations of Examples 2 to 6 and 8 to 10, Reference Examples 1 and 7, and Comparative Examples 1 to 12, an overall evaluation of tackiness and moistness when applying the foundation to the skin was conducted as per the following 3 grades. The results are shown in Tables 1 and 2.

### -Evaluation criteria-

A: Excellent with respect to all sensations
B: Overall, ordinary level of sensations
C: Poor with respect to all sensations

As shown in Tables 1 and 2, it is understood that the liquid foundations of Examples 2 to 6 and 8 to 10, and Reference Examples 1 and 7,, which include the components (a) to (d), are superior in all of the evaluations regarding alleviating yellow dulling, dissolution property (color unevenness), staining of skin, viscosity stability (dispersion stability), and use sensation (tackiness and moistness).

Here, in order to note in detail the effect of the water-soluble calcium salt contained as the component (d), the liquid foundations obtained in Example 3 and Comparative Example 4 are used as examples, and explanation is made by comparing these examples.

Referring to Table 1, the components of both Example 3 and Comparative Example 4 are the same except that Example 3 contains calcium chloride in the C phase while Comparative Example 4 contains sodium citrate instead of calcium chloride. However, in comparison with Comparative Example 4, which contains sodium citrate, it is understood that Example 3 is superior to Comparative Example 4 regarding dissolution property and staining of skin, and exhibits much higher viscosity stability compared with Comparative Example 4.

Details of the measured values of viscosity and the viscosity difference of each liquid foundation obtained in Example 3 and Comparative Example 4 are shown in Table 3 below.

**[Table 3]**

| | Example 3 | Comparative Example 4 |
|---|---|---|
| Day 1 | 2300mPa·s | 18000mPa·s |
| 4 weeks | 2400mPa·s | 28000mPa·s |
| Viscosity Difference | 100mPa·s | 1000mPa·s |

As shown in Table 3, in comparison with Comparative Example 4 including sodium citrate, it is understood that Example 3 including calcium chloride has viscosity stability that is ten times superior to that of Comparative Example 4. This is a surprising result.

The foregoing indicates that, as shown by Example 3, the inclusion of calcium chloride as the component (c) together with the components (a) and (b) can provide a liquid foundation which does not lead to color unevenness, which has good suppression of staining of skin, and which has superior viscosity stability.

Although the present invention has been described in detail with reference to Example 3, it is needless to say that the above description also applies to the other embodiments.

Further, the spectral reflectance of the apparent color with respect to each of the liquid foundations obtained in Example 3 and Comparative Example 2 was measured by a conventional method using a spectrophotometer (trade name: SPECTROLINO, manufactured by Gretag Machbeth). The results are shown in Figure 1.

As shown in Figure 1, it is shown that with respect to the liquid foundation of Example 3, the reflectance spectrum of the apparent color has a local minimal value in a wavelength region near 570 nm. On the other hand, it is shown with respect to the liquid foundation of Comparative Example 2, that the reflectance spectrum of the apparent color does not have a minimal value in a wavelength region between 500 nm and 600 nm.

## Claims

1. An emulsion-type base makeup cosmetic, comprising the following components (a), (b), (c), and (d):
(a) a powdered pigment comprising a red composite pigment in which lithol rubine BCA is intercalated into an inorganic substance;
(b) powdered pigments comprising an extender pigment, a coloring pigment, and a pearl pigment, and which are different from the component (a);
(c) an oily material; and
(d) a water-soluble calcium salt which is at least one selected from calcium chloride, calcium lactate, or calcium acetate.

2. The emulsion-type base makeup cosmetic according claim 1, wherein a content of the component (d) is in an amount of from 0.1% by mass to 5% by mass with respect to a total mass of the base makeup cosmetic.

3. The emulsion-type base makeup cosmetic according to either claim 1 or2, wherein a reflectance spectrum of an apparent color of the base makeup cosmetic has a local minimal value in a wavelength region of from 500 nm to 600 nm.

4. The emulsion-type base makeup cosmetic according to any one of claims 1 to 3, wherein the pearl pigment is included in an amount of from 5% by mass to 30% by mass with respect to a total mass of the component (b).

5. A method of producing an emulsion-type base makeup cosmetic, the method comprising:
preparing an oil phase wherein the oil phase comprises (a) a powdered pigment comprising a red composite pigment in which lithol rubine BCA is intercalated into an inorganic substance; (b) powdered pigments comprising an extender pigment, a coloring pigment, and a pearl pigment, and which are different from the component (a); and (c) an oily material, and
mixing the oil phase with an aqueous phase that comprises (d) a water-soluble calcium salt which is at least one selected from calcium chloride, calcium lactate, or calcium acetate, to form an emulsion.

6. The method of producing an emulsion-type base makeup cosmetic according to claim 5 wherein a content of the component (d) is in an amount of from 0.1% by mass to 5% by mass with respect to a total mass of the base makeup cosmetic.

## Patentansprüche

1. Basismakeup-Kosmetik vom Emulsionstyp umfassend die folgenden Komponenten (a), (b), (c) und (d):
(a) ein pulverförmiges Pigment umfassend ein rotes Verbundpigment, in dem Lithol Rubin BCA in eine anorganische Substanz interkaliert ist;
(b) pulverförmige Pigmente umfassend ein Streckpigment, ein farbgebendes Pigment und ein Perlglanzpigment, die unterschiedlich zur Komponente (a) sind;
(c) ein öliges Material; und
(d) ein wasserlösliches Calciumsalz, das mindestens eines, ausgewählt aus Calciumchlorid, Calciumlaktat und Calciumacetat ist.

2. Basismakeup-Kosmetik vom Emulsionstyp nach Anspruch 1, wobei ein Gehalt an Komponente (d) eine Menge von 0,1 Masse% bis 5 Masse%, bezogen auf eine Gesamtmasse der Basismakeup-Kosmetik, ist.

3. Basismakeup-Kosmetik vom Emulsionstyp nach entweder Anspruch 1 oder 2, wobei ein Reflexionsspektrum einer sichtbaren Farbe der Basismakeup-Kosmetik in einem Wellenlängenbereich von 500 nm bis 600 nm ein lokales Minimum aufweist.

4. Basismakeup-Kosmetik vom Emulsionstyp nach einem der Ansprüche 1 bis 3, wobei das Perlglanzpigment in einer Menge von 5 Masse% bis 30 Masse%, bezogen auf eine Gesamtmasse an der Komponente (b), enthalten ist.

5. Verfahren zur Herstellung einer Basismakeup-Kosmetik vom Emulsionstyp, das Verfahren umfassend:
Herstellen einer Ölphase, wobei die Ölphase (a) ein pulverförmiges Pigment umfassend ein rotes Verbundpigment, in dem Lithol Rubin BCA in eine anorganische Substanz interkaliert ist und (b) pulverförmige Pigmente enthaltend ein Streckpigment, ein farbgebendes Pigment und ein Perlglanzpigment, die unterschiedlich zur Komponente (a) sind; und (c) ein öliges Material umfasst, und
Mischen der Ölphase mit einer Wasserphase, die (d) ein wasserlösliches Calciumsalz umfasst, das mindestens eines, ausgewählt aus Calciumchlorid, Calciumlaktat und Calciumacetat ist, um eine Emulsion zu bilden.

6. Verfahren zur Herstellung einer Basismakeup-Kosmetik vom Emulsionstyp nach Anspruch 5, wobei ein Gehalt an der Komponente (d) eine Menge von 0,1 Masse% bis 5 Masse%, bezogen auf eine Gesamtmasse der Basismakeup-Kosmetik, ist.

## Revendications

1. Cosmétique de maquillage de base de type émulsion, comprenant les composants suivants (a), (b), (c), et (d) :
(a) un pigment en poudre comprenant un pigment composite rouge où de la Lithol Rubine BCA est intercalée dans une substance inorganique ;
(b) des pigments en poudre comprenant un pigment de charge, un pigment de coloration, et un pigment nacré, lesquels sont différents du composant (a) ;
(c) une matière huileuse, et
(d) un sel de calcium soluble dans l'eau, lequel est au moins un élément sélectionné parmi du chlorure de calcium, du lactate de calcium, ou de l'acétate de calcium.

2. Cosmétique de maquillage de base de type émulsion selon la revendication 1, dans lequel une teneur du composant (d) se présente dans une quantité allant de 0,1 % en masse à 5 % en masse par rapport à une masse totale du cosmétique de maquillage de base.

3. Cosmétique de maquillage de base de type émulsion selon la revendication 1 ou 2, dans lequel un spectre de facteur de réflexion d'une couleur apparente du cosmétique de maquillage de base présente une valeur minimale locale dans une région de longueurs d'onde allant de 500 nm à 600 nm.

4. Cosmétique de maquillage de base de type émulsion selon l'une quelconque des revendications 1 à 3, dans lequel le pigment nacré est inclus en une quantité allant de 5 % en masse à 30 % en masse par rapport à une masse totale du cosmétique de maquillage de base (b).

5. Procédé de production d'un cosmétique de maquillage de base de type émulsion, le procédé comprenant les étapes consistant à :
préparer une phase huileuse, dans lequel la phase huileuse comprend (a) un pigment en poudre comprenant un pigment composite rouge où de la Lithol Rubine BCA est intercalée dans une substance inorganique ; (b) des pigments en poudre comprenant un pigment de charge, un pigment de coloration, et un pigment nacré, lesquels sont différents du composant (a), et (c) une matière huileuse, et
mélanger la phase huileuse avec une phase aqueuse, laquelle comprend (d) un sel de calcium soluble dans l'eau, lequel est au moins un élément sélectionné parmi du chlorure de calcium, du lactate de calcium, ou de l'acétate de calcium, afin de former une émulsion.

6. Procédé de production d'un cosmétique de maquillage de base de type émulsion selon la revendication 5, dans lequel une teneur du composant (d) se présente dans une quantité allant de 0,1 % en masse à 5 % en masse par rapport à une masse totale du cosmétique de maquillage de base.
